# EUROPEAN PATENT APPLICATION

(11) **EP 4 574 096 A1**
(43) Date of publication of application: **25.06.2025**
(21) Application number: 23218801.1
(22) Date of filing: 20.12.2023
(51) Int. Cl.: A61F 2/30, A61F 2/46

(54) **TRIAL AUGMENT AND SET OF TRIAL AUGMENTS FOR A BONE IMPLANT**

(71) Applicant: Aesculap AG, 78532 Tuttlingen (DE)
(72) Inventor: NONNENMANN, Martin, 78573 Wurmlingen (DE); ZOUAGHI, Housseyn, 52000 Chaumont (FR); SNAUWAERT, Eliott, 52000 Chaumont (FR)
(74) Representative: DREISS Patentanwälte PartG mbB

(57) **Abstract**

The invention relates to a trial augment (100, 600) for a bone implant and to a set of the trial augments (100, 600), wherein the trial augment (100, 600) is configured to augment the bone implant in a first direction (101, 601), in particular in a proximal or distal direction, wherein the trial augment (100, 600) comprises a first connection element (104, 604) that is configured for connecting the trial augment (100, 600) to a device, in particular the bone implant or another trial augment, wherein the trial augment (100, 600) comprises a second connection element (106, 606) that is configured to connect to another trial augment to further augment the bone implant in the first direction.

## Description

The invention relates to a trial augment and a set of trial augments for a bone implant.

Trial augments are used to determine the size of an augment that is required to augment a bone implant in accordance with a size of a bone defect of a patient receiving the bone implant.

A trial augment for a bone implant is configured to augment the bone implant in a first direction, in particular in a proximal or distal direction, wherein the trial augment comprises a first connection element that is configured for connecting the trial augment to a device, in particular the bone implant or another trial augment, wherein the trial augment comprises a second connection element that is configured to connect to another trial augment to further augment the bone implant in the first direction. The trial augment is stackable. This enables giving different thicknesses of an augmentation of the bone implant a trial with either the trial augment alone or with a stack of trial augments.

The trial augment may comprise a body for augmenting the bone implant, wherein the first connection element protrudes on a distal side and the second connection element recesses on a proximal side of the body, or wherein the first connection element recesses on a proximal side and the second connection element protrudes on a distal side of the body. This arrangement of the connection elements enables receiving the protruding connection element of one trial augment in the body of another trial augment.

Preferably, the first connection element extends in a second direction, in particular in posterior to anterior direction or essentially in posterior to anterior direction of the trial augment, and the second connection element extends in the second direction or a third direction. Arranging both connecting elements to extend in the second direction means, the first connection element and the second connection element are arranged for sliding the protruding connection element of the trial augment into the recessing connection element of the other trial augment in the same direction as the direction that is used for sliding the recessing connection element of the trial augment over the protruding element of the other trial augment. Using the second direction and the third direction provide different sliding directions on opposite sides of the trial augment.

According to an example, the first connection element comprises a first member of a linear slide, in particular of a dovetail slide, that has a cross-section for interlocking interaction with a second member of the linear slide provided by the device or the other trial augment. This allows to slide the first member of the trial augment with respect to the second member of the other augment to provide the stack. This trial augment is stackable on one side with the other trial augment having the matching second member.

Preferably, the first member is configured to slide in the linear slide. This enables a connection of the trial augments that is guided by the linear slide.

According to an example, the trial augment comprises a spring assembly, in particular a spring sleeve, wherein the first member comprises a wall with an opening for the spring assembly, wherein the spring assembly is arranged in the opening, wherein the spring assembly is configured to protrude from the first member, wherein the spring assembly is configured to be pushed into the opening flush with the wall or essentially flush with the wall. The spring assembly is configured to be pushed in the opening when the second member and the first member engage. The spring assembly is configured to protrude into a housing in the second member that is configured to receive a part of the spring assembly. This prevents a movement of the trial augments in the posterior to anterior direction, when the spring assembly protrudes into the housing.

Preferably, the spring assembly is configured protrude from the first member at an angle with respect to the wall that is 90° or 45° or between 90 % and 45°.

According to an example, the second connection element comprises a second member of a linear slide, in particular of a dovetail slide, that is configured to lock into a first member of a linear slide of the other trial augment, wherein the second member is configured to slide in the linear slide. This enables a guided connection of the trial augments.

Preferably, the second member comprises a housing that is configured to receive a part of a spring assembly, in particular a part of a sleeve of a spring sleeve, that protrudes from a first member of the other trial augment.

According to an example, the second member has a cross-section for interlocking interaction with a first member of the linear slide provided by the other trial augment, in particular a groove with an undercut or a dovetail groove. This allows to slide the second member of the trial augment with respect to the first member of the other augment to provide the stack. This trial augment is stackable on the other side with the other augment having the matching first member. A trial augment having the first member and the second member with the respective cross-section are stackable on both sides with other trial augments having the matching first and second member.

A set of trial augments comprises at least two of the trial augments.

The set of trial augments preferably comprises at least two trial augments that have bodies that extent in the direction to different extends. This reduces the space that is needed in a tray. In particular a biggest thickness of conventional sets of trial augments is used only rarely, in cases of revision with significant bone defects. Consequently, the amount of trial augments in the tray is reduced because at least the biggest trial augment that is used in rare cases is avoided in the tray.

Further embodiments are derived from the following description and the drawing. In the drawing:
- Fig. 1: schematically depicts a perspective view of a first embodiment of a trial augment,
- Fig. 2: schematically depicts a bottom view of the first embodiment,
- Fig. 3: schematically depicts an enlarged view of a part of the bottom view of the first embodiment,
- Fig. 4: schematically depicts two trial augments according to the first embodiment,
- Fig. 5: schematically depicts a stack of the two trial augments according to the first embodiment,
- Fig. 6: schematically depicts a perspective view of a second embodiment of a trial augment,
- Fig. 7: schematically depicts a bottom view of the second embodiment,
- Fig. 8: schematically depicts an enlarged view of a part of the bottom view of the second embodiment,
- Fig. 9: schematically depicts two trial augments according to the second embodiment,
- Fig. 10: schematically depicts a stack of the two trial augments according to the second embodiment.

Trial augments are provided as instrument for augmenting a bone implant to a required extend. The trial augments described herein are stackable with each other. This provides a surgeon with the ability to obtain a higher thickness trial augment by combining two or more trial augments together. The consequence of this is a space saving of the instruments in a tray.

The bone implant may be a tibia implant or a femur implant. The trial augment may be configured to augment the tibia implant or the femur implant.

In the example the two trial augments are connected with a linear slide, in particular of a dovetail slide. When two trial augments are connected, they need to be stable for avoiding an unwanted disconnection. According to an example, during the connection of a first trial augment to a second trial augment, a spring assembly of the first trial augment goes in a housing provided in a groove of the linear slide of the second trial augment.

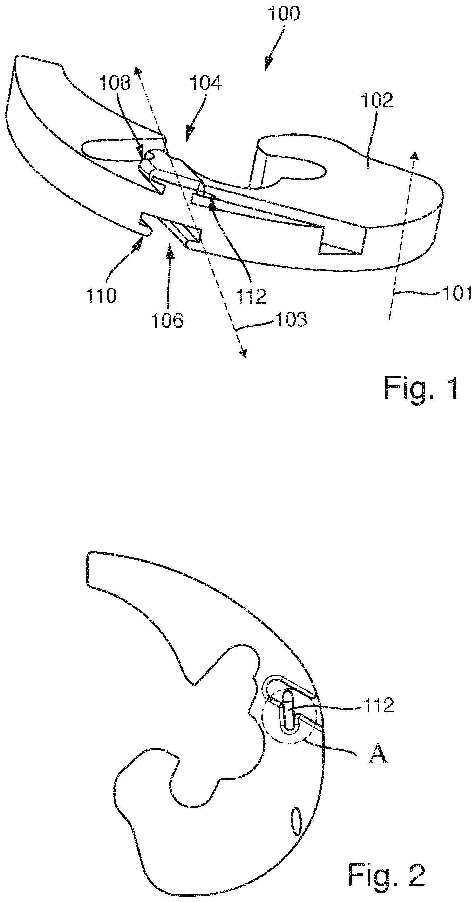

Figure 1 schematically depicts a perspective view of a first embodiment 100 of the trial augment. The trial augment according to the first embodiment 100 is configured to augment the tibia implant in a distal direction 101.

The trial augment according to the first embodiment 100 comprises a body 102. The body 102 is configured to extend the tibia implant in the distal direction 101.

The trial augment according to the first embodiment 100 comprises a first connection element 104 that is configured for connecting the trial augment to a device.

The device may be the tibia implant or another trial augment according to the first embodiment 100.

The trial augment according to the first embodiment 100 optionally comprises a second connection element 106 that is configured to connect to another trial augment according to the first embodiment 100 to further augment the tibia implant in the distal direction 101.

The first connection element 104 according to the first embodiment 100 protrudes from the body 102. The first connection element 104 may be a groove in the body 102. The second connection element 106 according to the first embodiment 100 is groove in the body 102. The second connection element 106 may protrude from the body 102. The first connection element 104 is arranged on a distal side of the body 102 of the augment according to the first embodiment 100. The second connection element 106 is arranged on a proximal side of the body 102 of the augment according to the first embodiment 100.

The first connection element 104 and the second connection element 106 extend in posterior to anterior direction 103 or essentially in posterior to anterior direction of the trial augment 100.

The first connection element 104 comprises a first member 108 of the linear slide, in particular of the dovetail slide.

The first member 108 according to the first embodiment 100 is configured to slide in the linear slide. Preferably, the first member 108 is configured to slide in the second direction 103 according to the first embodiment 100.

The first member 108 according to the first embodiment 100 may be configured to slide essentially in posterior to anterior direction 103 and/or essentially in anterior to posterior direction of the trial augment according to the first embodiment 100.

The first member 108 according to the first embodiment 100 has a cross-section for interlocking interaction with a second member of the linear slide provided by the device or the other trial augment according to the first embodiment 100.

The second connection element 104 comprises a second member 110 of a linear slide, in particular of the dovetail slide, that is configured for connecting to another trial augment according to the first embodiment 100.

The second member 110 according to the first embodiment 100 is configured to lock into a first member of the linear slide of the other trial augment.

The second member 110 according to the first embodiment 100 is configured to slide in the linear slide. The second member 110 may be configured to slide in the second direction 103 or the third direction according to the first embodiment 100.

The second member 110 according to the first embodiment 100 may be configured to slide essentially in posterior to anterior direction 103 and/or essentially in anterior to posterior direction of the trial augment according to the first embodiment 100.

The second member 110 may have a cross-section for interlocking interaction with a first member 108 of the linear slide provided by the other trial augment.

The cross-section may be that of a groove with an undercut or a dovetail groove.

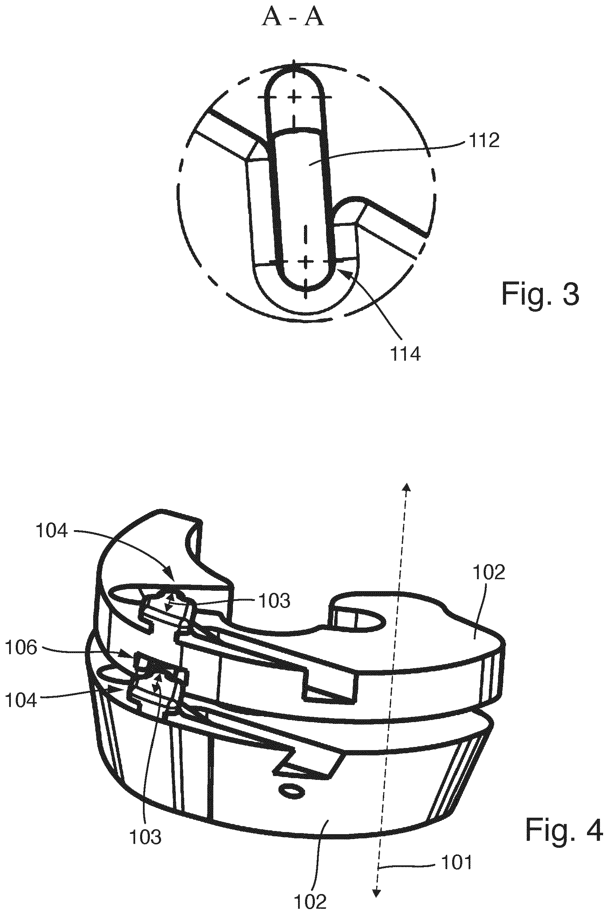

The trial augment according to the first embodiment 100 comprises a spring assembly 112. The spring assembly 112 is described with reference to figures 2 and 3.

Figure 2 schematically depicts a bottom view of the first embodiment 100. Figure 3 schematically depicts an enlarged view of a part "A" of the bottom view of the first embodiment.

The first member 108 according to the first embodiment 100 comprises an opening 114 for the spring assembly 112. The opening 114 may be provided in a wall of the first member 108.

The spring assembly 112 is arranged in the opening 114. The spring assembly 112 is configured to protrude from the first member 108. The spring assembly 112 may be configured protrude from the first member 108 at an angle with respect to the wall. The angle is for example is 90° or 45° or between 90 % and 45°.

The spring assembly 112 is configured to be pushed into the opening 114. The spring assembly 112 is for example configured to be pushed into the opening flush with the wall or essentially flush with the wall.

The second member 110 may comprise a housing that is configured to receive a part of a spring assembly 112, in particular a part of a sleeve of a spring sleeve, that protrudes from a first member of the other trial augment.

Figure 4 schematically depicts a perspective view of a set of two trial augments according to the first embodiment 100 before assembly to a stack.

The first connection element 104 of one of the trial augments according to the first embodiment 100 and the second member 106 of the other trial augment according to the first embodiment 100 are aligned with each other in the posterior to anterior direction 103. The two exemplary trial augments according to the first embodiment 100 have bodies 102 that extent in the first direction 101 to different extends. The stack may comprise trial augments of same height in the first direction 101.

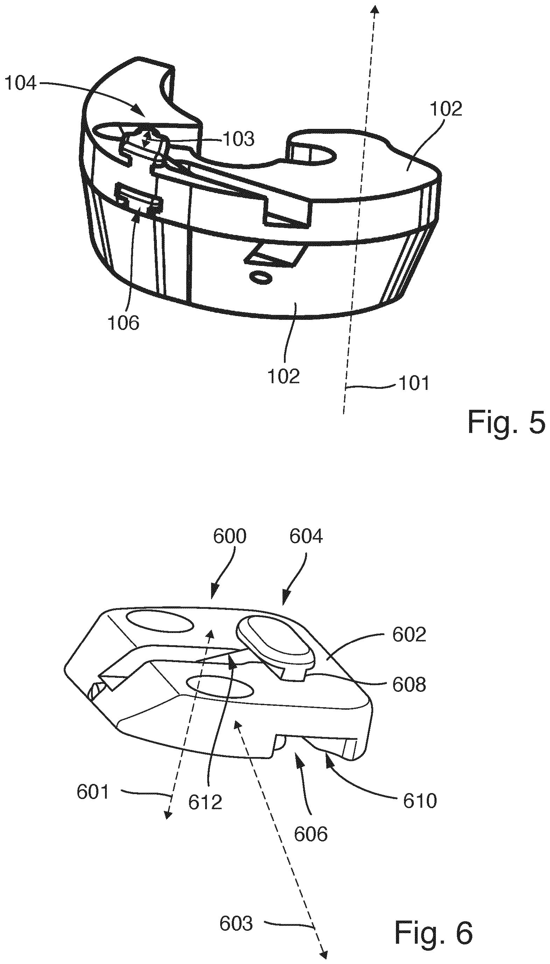

Figure 5 schematically depicts a stack of the two trial augments according to the first embodiment 100 i.e., after assembly to the stack.

The stack may comprise more than two of the trial augments according to the first embodiment 100.

The bodies 102 of the different trial augments according to the first embodiment 100 may differ in their shape and/or the extent in the second direction 103 or the third direction.

Figure 6 schematically depicts a perspective view of a second embodiment 600 of the trial augment. The trial augment according to the second embodiment 600 is configured to augment the femur implant in a proximal direction 601.

The trial augment according to the second embodiment 600 comprises a body 602. The body 602 is configured to extend the femur implant in the proximal direction 601.

The trial augment according to the second embodiment 600 comprises a first connection element 604 that is configured for connecting the trial augment to a device.

The device may be the femur implant or another trial augment according to the second embodiment 600.

The trial augment according to the second embodiment 600 optionally comprises a second connection element 606 that is configured to connect to another trial augment according to the second embodiment 600 to further augment the femur implant in the proximal direction 601.

The first connection element 604 according to the second embodiment 600 protrudes from the body 602. The first connection element 604 may be a groove in the body 602. The second connection element 606 according to the second embodiment 600 is groove in the body 602. The second connection element 606 may protrude from the body 602. The first connection element 604 is arranged on a proximal side of the body 602 of the augment according to the second embodiment 600. The second connection element 606 is arranged on a distal side of the body 602 of the augment according to the second embodiment 600.

The first connection element 604 and the second connection element 606 extend in posterior to anterior direction 603 or essentially in posterior to anterior direction of the trial augment 600.

The first connection element 604 comprises a first member 608 of the linear slide, in particular of the dovetail slide.

The first member 608 according to the second embodiment 600 is configured to slide in the linear slide. Preferably, the first member 608 is configured to slide in the second direction 603 according to the second embodiment 600.

The first member 608 according to the second embodiment 600 may be configured to slide essentially in posterior to anterior direction 603 and/or essentially in anterior to posterior direction of the trial augment according to the second embodiment 600.

The first member 608 according to the second embodiment 600 has a cross-section for interlocking interaction with a second member of the linear slide provided by the device or the other trial augment according to the second embodiment 600.

The second connection element 604 comprises a second member 610 of a linear slide, in particular of the dovetail slide, that is configured for connecting to another trial augment according to the second embodiment 600.

The second member 610 according to the second embodiment 600 is configured to lock into a first member of the linear slide of the other trial augment.

The second member 610 according to the second embodiment 600 is configured to slide in the linear slide. The second member 610 may be configured to slide in the second direction 603 or the third direction according to the second embodiment 600.

The second member 610 according to the second embodiment 600 may be configured to slide essentially in posterior to anterior direction 603 and/or essentially in anterior to posterior direction of the trial augment according to the second embodiment 600.

The second member 610 may have a cross-section for interlocking interaction with a first member 608 of the linear slide provided by the other trial augment.

The cross-section may be that of a groove with an undercut or a dovetail groove.

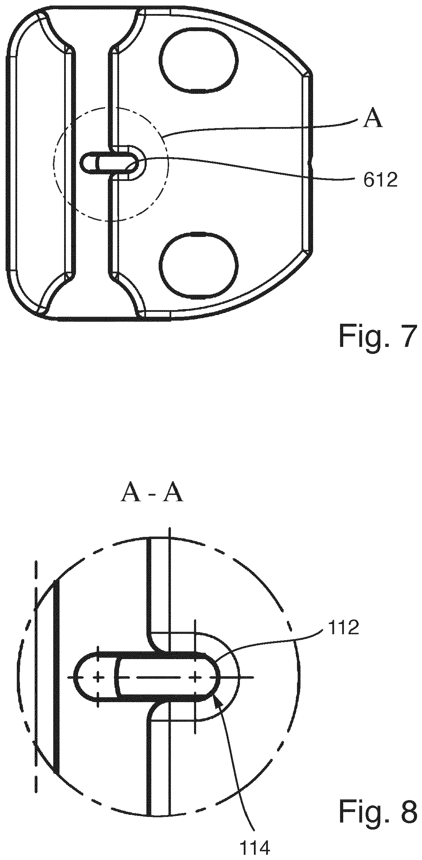

The trial augment according to the second embodiment 600 comprises a spring assembly 612. The spring assembly 612 is described with reference to figures 7 and 8.

Figure 7 schematically depicts a bottom view of the second embodiment 600. Figure 8 schematically depicts an enlarged view of a part "A" of the bottom view of the second embodiment 600.

The first member 608 according to the second embodiment 600 comprises an opening 614 for the spring assembly 612. The opening 614 may be provided in a wall of the first member 608.

The spring assembly 612 is arranged in the opening 614. The spring assembly 612 is configured to protrude from the first member 608. The spring assembly 612 may be configured protrude from the first member 608 at an angle with respect to the wall. The angle is for example is 90° or 45° or between 90 % and 45°.

The spring assembly 612 is configured to be pushed into the opening 614. The spring assembly 612 is for example configured to be pushed into the opening flush with the wall or essentially flush with the wall.

The second member 610 may comprise a housing that is configured to receive a part of a spring assembly 612, in particular a part of a sleeve of a spring sleeve, that protrudes from a first member of the other trial augment.

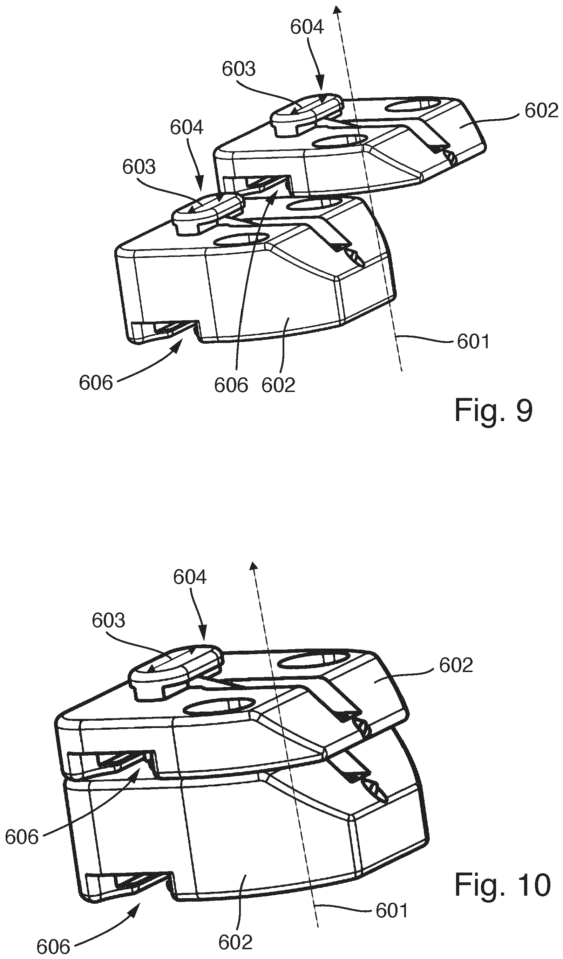

Figure 9 schematically depicts a perspective view of a set of two trial augments according to the second embodiment 600 before assembly to a stack.

The first connection element 604 of one of the trial augments according to the second embodiment 600 and the second member 606 of the other trial augment according to the second embodiment 600 are aligned with each other in the posterior to anterior direction 603. The two exemplary trial augments according to the second embodiment 600 have bodies 602 that extent in the first direction 601 to different extends. The stack may comprise trial augments of same height in the first direction 601.

Figure 10 schematically depicts a stack of the two trial augments according to the second embodiment 600 i.e., after assembly to the stack.

The stack may comprise more than two of the trial augments according to the second embodiment 600.

The bodies 602 of the different trial augments according to the second embodiment 600 may differ in their shape and/or the extent in the second direction 603 or the third direction.

## Claims

1. Trial augment (100, 600) for a bone implant, **characterized in that** the trial augment (100, 600) is configured to augment the bone implant in a first direction (101, 601), in particular in a proximal or distal direction, wherein the trial augment (100, 600) comprises a first connection element (104, 604) that is configured for connecting the trial augment (100, 600) to a device, in particular the bone implant or another trial augment, wherein the trial augment (100, 600) comprises a second connection element (106, 606) that is configured to connect to another trial augment to further augment the bone implant in the first direction.

2. The trial augment (100, 600) according to claim 1, **characterized in that** the trial augment comprises a body (102, 602) for augmenting the bone implant, wherein the first connection element (104, 604) protrudes on a distal side and the second connection element (106, 606) recesses on a proximal side of the body (102, 602) or wherein the first connection element (104, 604) recesses on a proximal side and the second connection element (106, 606) protrudes on a distal side of the body (102, 602).

3. The trial augment (100, 600) according to one of the previous claims, **characterized in that** the first connection element (104, 604) extends in a second direction (103, 603), in particular a posterior to anterior direction or essentially in posterior to anterior direction of the trial augment (100, 600), and the second connection element (106, 606) extends in the second direction (103, 603) or a third direction.

4. The trial augment (100, 600) according to one of the preceding claims, **characterized in that** the first connection element (104, 604) comprises a first member (108, 608) of a linear slide, in particular of a dovetail slide, that has a cross-section for interlocking interaction with a second member (110, 610) of the linear slide provided by the device or the other trial augment.

5. The trial augment (100, 600) according to claim 4, **characterized in that** the first member (108, 608) is configured to slide in the linear slide.

6. The trial augment (100, 600) according to one of the preceding claims, **characterized in that** the trial augment (100, 600) comprises a spring assembly (112, 612), in particular a spring sleeve, wherein the first member (108, 608) comprises a wall with an opening (114, 614) for the spring assembly (112, 612), wherein the spring assembly is arranged in the opening (114, 614), wherein the spring assembly (112, 612) is configured to protrude from the first member (108, 608), wherein the spring assembly (112, 612) is configured to be pushed into the opening (114, 614) flush with the wall or essentially flush with the wall.

7. The trial augment (100, 600) according to claim 6, **characterized in that** the spring assembly (112, 612) is configured protrude from the first member (108, 608) at an angle with respect to the wall that is 90° or 45° or between 90 % and 45°.

8. The trial augment (100, 600) according to one of the preceding claims, **characterized in that** the second connection element (104, 604) comprises a second member (110, 610) of a linear slide, in particular of a dovetail slide, that is configured to lock into a first member (108, 608) of a linear slide of the other trial augment, wherein the second member (110, 610) is configured to slide in the linear slide.

9. The trial augment (100, 600) according to claim 8, **characterized in that** the second member (110, 610) has a cross-section for interlocking interaction with a first member (108, 608) of the linear slide provided by the other trial augment, in particular a groove with an undercut or a dovetail groove.

10. The trial augment (100, 600) according to one of the claims 8 to 9, **characterized in that** the second member (110, 610) comprises a housing that is configured to receive a part of a spring assembly (112, 612), in particular a part of a sleeve of a spring sleeve, that protrudes from a first member of the other trial augment.

11. A set of trial augments (100, 600), **characterized in that** the set of trial augments (100, 600) comprises at least two trial augments according to one of the claims 1 to 10.

12. The set of trial augments (100, 600) according to claim 11, **characterized in that** the set comprises at least two trial augments (100, 600) that have bodies (102, 602) that extent in the first direction (101, 601) to different extends.
